# EUROPÄISCHE PATENTSCHRIFT

(11) **EP 1 261 593 B1**
(45) Veröffentlichungstag und Bekanntmachung des Hinweises auf die Patenterteilung: **27.08.2003**
(21) Anmeldenummer: 01916989.5
(22) Anmeldetag: 13.02.2001
(51) Int. Cl.: C07D 277/60, A61K 31/428, A61P 3/04

(54) **8,8A-DIHYDRO-INDENO 1,2-D]THIAZOL-DERIVATE, DIE IN 2-STELLUNG EINEN SUBSTITUENTEN MIT EINER SULFONAMIDSTRUKTUR ODER SULFONSTRUKTUR TRAGEN; VERFAHREN ZU IHRER HERSTELLUNG UND IHRE VERWENDUNG ALS ARZNEIMITTEL**
8,8A-DIHYDRO-INDENO 1,2-D]THIAZOLE DERIVATIVES WITH A SULPHONAMIDO OR SULPHONO SUBSTITUENT IN THE 2 POSITION, A METHOD FOR PRODUCTION THEREOF AND USE THEREOF AS A MEDICAMENT
DERIVES DE 8,8A-DIHYDRO-INDENO 1,2-D]THIAZOL, PORTANT EN POSITION 2 UN SUBSTITUANT DE STRUCTURE SULFONAMIDE OU SULFONE; LEUR PROCEDE DE PRODUCTION ET LEUR UTILISATION COMME MEDICAMENT

(30) Priorität: 26.02.2000 DE 10009311
(43) Veröffentlichungstag der Anmeldung: 04.12.2002
(73) Patentinhaber: Aventis Pharma Deutschland GmbH, 65929 Frankfurt am Main (DE)
(72) Erfinder: JAEHNE, Gerhard, 65929 Frankfurt (DE); LANG, Hans-Jochen, 65719 Hofheim (DE); GOSSEL, Matthias, 65719 Hofheim (DE); BICKEL, Martin, 61348 Bad Homburg (DE)
(86) Internationale Anmeldenummer: EP0101553
(87) Internationale Veröffentlichungsnummer: WO01062747

(56) Entgegenhaltungen:
- WO-A-00/51997
- US-A- 4 174 397

## Beschreibung

8,8a-Dihydro-indeno[1,2-d]thiazol-Derivate, die in 2-Stellung einen Substituenten mit einer Sulfonamidstruktur oder Sulfonstruktur tragen; Verfahren zu ihrer Herstellung und ihre Verwendung als Arzneimittel

Die Erfindung betrifft polycyclische Dihydrothiazole sowie deren physiologisch verträgliche Salze und physiologisch funktionelle Derivate.

Es sind bereits Thiazolidinderivate mit anorektischer Wirkung im Stand der Technik beschrieben (Österreichisches Patent Nr. 365181).

Der Erfindung lag die Aufgabe zugrunde, weitere Verbindungen zur Verfügung zu stellen, die eine therapeutisch verwertbare anorektische Wirkung entfalten.

Die Erfindung betrifft daher Verbindungen der Formel I, 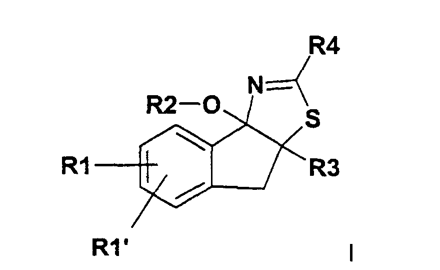 worin bedeuten
- R1, R1': unabhängig voneinander H, F, Cl, Br, J, CF₃, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können, oder ein Wasserstoff durch OH, OC(O)CH₃, OC(O)H, O-CH₂-Ph, NH₂, NH-CO-CH₃ oder N(COOCH₂Ph)₂ ersetzt sein kann; SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; NH₂, NH-(C₁-C₆)-Alkyl, N((C₁-C₆)-Alkyl)₂, NH(C₁-C₇)-Acyl, Phenyl, Biphenyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Biphenyl-, Naphthyl-, Pyridyl-, Furanyloder Thienylringe jeweils ein bis 3-fach substituiert sein können mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C1-C₆)-Alkyl, CONH₂; 1,2,3-Triazol-5-yl, wobei der Triazolring in 1-, 2- oder 3-Stellung mit Methyl oder Benzyl substituiert sein kann; Tetrazol-5-yl, wobei der Tetrazolring in 1- oder 2-Stellung mit Methyl oder Benzyl substituiert sein kann;
- R2: H, (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, C(O)-(C₁-C₆)-Alkyl, C(O)-(C₃-C₆)-Cycloalkyl, C(O)-(CH₂)ₙ-Phenyl, C(O)-(CH₂)ₙ-Thienyl, C(O)-(CH₂)ₙ-Pyridyl, C(O)-(CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann;
- R3: H, (C₁-C₆)-Alkyl, F, CN, N₃, O-(C₁-C₆)-Alkyl, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C₁-C₆)-Alkyl substituiert sein kann; OC(O)CH₃, (C₂-C₆)-Alkinyl, (C₂-C₆)-Alkenyl, COO(C₁-C₆)-Alkyl, C(O)OH, C(O)NH₂, C(O)NHCH₃, C(O)N(CH₃)₂;
- R4: (CH₂)n-R5, wobei n = 0 - 6 sein kann;
- R5: Phenyl, Biphenyl, 1- oder 2-Naphthyl, 2-, 3- oder 4-Pyridyl, 2- oder 3-Thienyl, 2- oder 3-Furyl, 2-, 4- oder 5-Thiazolyl, 2-, 4- oder 5-Oxazolyl, 1-Pyrazolyl, 3- oder 5-Isoxazolyl, 2- oder 3-Pyrrolyl, 2- oder 3-Pyridazinyl, 2-, 4- oder 5-Pyrimidinyl, 2-Pyrazinyl, 2-(1,3,5-Triazinyl), 2- oder 5-Benzimidazolyl, 2-Benzothiazolyl, 1,2,4-Triazol-3-yl, 1,2,4-Triazol-5-yl, Tetrazol-5-yl, Indol-3-yl, Indol-5-yl oder N-Methyl-imidazol-2-, 4- oder -5-yl;
- und R5: substituiert ist mit NH-SO₂-(C₁-C₆)-Alkyl, NH-SO₂-Phenyl, wobei der Phenylring bis zu zweifach mit F, Cl, CN, OH, (C₁-C₆)-Alkyl, O-(C₁-C₆)-Alkyl, CF₃, COOH, COO(C₁-C₆)-Alkyl, CONH₂ substituiert sein kann; (CH₂)n-SO₂-(C₁-C₆)-Alkyl, wobei n = 1 - 6 sein kann, (CH₂)m-SO₂-NH₂ , (CH₂)m-SO₂-NH-(C₁-C₆)-Alkyl, (CH₂)m-SO₂-N((C₁-C₆)-Alkyl)₂ oder (CH₂)m-SO₂-N(=CH-N(CH₃)₂), wobei m = 0 - 6 sein kann;
- und R5: gegebenenfalls zusätzlich mit F, Cl, Br, OH, CF₃, NO₂, CN, OCF₃, O-(C₁-C₆)-Alkyl, S-(C₁-C₆)-Alkyl, SO-(C₁-C₆)-Alkyl, (C₁-C₆)-Alkyl, (C₃-C₆)-Cycloalkyl, COOH, COO(C₁-C₆)Alkyl, COO(C₃-C₆)Cycloalkyl, CONH₂, CONH(C₁-C₆)Alkyl, CON[(C₁-C₆)Alkyl]2, CONH(C₃-C₆)Cycloalkyl, NH₂, NH-CO-(C₁-C₆)-Alkyl, NH-CO-Phenyl, Pyrrolidin-1-yl, Morpholin-1-yl, Piperidin-1-yl, Piperazin-1-yl, 4-Methyl-piperazin-1-yl, (CH₂)n-Phenyl, O-(CH₂)n-Phenyl, S-(CH₂)n-Phenyl, SO₂-(CH₂)n-Phenyl, wobei n = 0-3 sein kann, substituiert sein kann;
sowie deren physiologisch verträgliche Salze und physiologisch funktionelle Derivate.

Bevorzugt sind Verbindungen der Formel I, worin bedeuten
- R1, R1': unabhängig voneinander H, F, Cl, Br, J, CF₃, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein Wasserstoff durch OH, OC(O)CH₃, OC(O)H, O-CH₂-Ph, NH₂, NH-CO-CH₃ oder N(COOCH₂Ph)₂ ersetzt sein kann; SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; NH₂, NH-(C₁-C₆)-Alkyl, N((C₁-C₆)-Alkyl)₂, NH(C₁-C₇)-Acyl, Phenyl, Biphenyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Biphenyl-, Naphthyl-, Pyridyl-, Furanyloder Thienylringe jeweils ein bis 3-fach substituiert sein können mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C1-C₆)-Alkyl, CONH₂;
1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;
- R2: H, (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, C(O)-(C₁-C₆)-Alkyl, C(O)-(C₃-C₆)-Cycloalkyl, C(O)-(CH₂)ₙ-Phenyl, C(O)-(CH₂)ₙ-Thienyl, C(O)-(CH₂)ₙ-Pyridyl, C(O)-(CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C₁-C₆)-Alkyl substituiert sein kann;
- R3: H, (C₁-C₆)-Alkyl, F, CN, N₃, O-(C₁-C₆)-Alkyl, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C₁-C₆)-Alkyl substituiert sein kann; OC(O)CH₃, (C₂-C₆)-Alkinyl, (C₂-C₆)-Alkenyl, COO(C1-C6)-Alkyl, C(O)OH, C(O)NH₂, C(O)NHCH₃, C(O)N(CH₃)₂;
- R4: (CH₂)n-R5, wobei n = 0 - 6 sein kann;
- R5: Phenyl, Biphenyl, 1- oder 2-Naphthyl, 2-, 3- oder 4-Pyridyl oder 2-oder 3-Thienyl sein kann;
- und R5: substituiert ist mit NH-SO₂-(C₁-C₆)-Alkyl, NH-SO₂-Phenyl, wobei der Phenylring bis zu zweifach mit F, Cl, CN, OH, (C₁-C₆)-Alkyl, O-(C₁-C₆)-Alkyl, CF₃, COOH, COO(C₁-C₆)-Alkyl, CONH₂ substituiert sein kann; (CH₂)n-SO₂-(C₁-C₆)-Alkyl, wobei n = 1 - 6 sein kann, (CH₂)m-SO₂-NH₂, (CH₂)m-SO₂-NH-(C₁-C₆)-Alkyl, (CH₂)m-SO₂-N((C₁-C₆)-Alkyl)₂ oder (CH₂)m-SO₂-N(=CH-N(CH₃)₂), wobei m = 0 - 6 sein kann;
- und R5: gegebenenfalls zusätzlich mit F, Cl, Br, OH, CF₃, CN, OCF₃, O-(C₁-C₆)-Alkyl, (C₁-C₆)-Alkyl, (C₃-C₆)-Cycloalkyl, COOH, COO(C₁-C₆)Alkyl, COO(C₃-C₆)Cycloalkyl, CONH₂, CONH(C₁-C₆)Alkyl, CON[(C₁-C₆)Alkyl]₂, NH₂, NH-CO-(C₁-C₆)-Alkyl, NH-CO-Phenyl, (CH₂)n-Phenyl, O-(CH₂)n-Phenyl, S-(CH₂)n-Phenyl, SO₂-(CH₂)n-Phenyl, wobei n = 0-3 sein kann, substituiert sein kann;
sowie deren physiologisch verträgliche Salze und physiologisch funktionelle Derivate.

Besonders bevorzugt sind Verbindungen der Formel I, worin bedeuten
- R1, R1': unabhängig voneinander H, F, Cl, Br, J, CF₃, NO₂, CN, COOH, COO(C₁-C₆)Alkyl, CONH₂, (C₁-C₆)-Alkyl, (C₂-C₆)-Alkenyl, (C₂-C₆)-Alkinyl, O-(C₁-C₆)-Alkyl, SO₂-NH₂, Phenyl, O-(CH₂)ₙ-Phenyl, wobei n = 0 - 6 sein kann, wobei die Phenylringe jeweils ein bis 3-fach substituiert sein können mit F, Cl, Br, J, OH, CF₃, NO₂, CN, OCF₃, O-(C₁-C₆)-Alkyl, (C₁-C₆)-Alkyl, NH₂, CONH₂;
- R2: H, (C₁-C₆)-Alkyl, (C₃-C₆)-Cycloalkyl, (CH₂)ₙ-Phenyl, C(O)-(C₁-C₆)-Alkyl, C(O)-(CH₂)ₙ-Phenyl, wobei n = 0 - 5 sein kann und worin Phenyl bis zu zweimal mit Cl, F, CN, CF₃, (C₁-C₃)-Alkyl, OH, O-(C1-C₆)-Alkyl substituiert sein kann;
- R3: H, (C₁-C₆)-Alkyl, F, (CH₂)ₙ-Phenyl, wobei n = 0 - 5 sein kann und worin Phenyl bis zu zweimal mit Cl, F, CN, CF₃, (C₁-C₃)-Alkyl, OH, O-(C₁-C₆)-Alkyl substituiert sein kann; COO(C₁-C₆)-Alkyl, C(O)OH, C(O)NH₂ ;
- R4: (CH₂)n-R5, wobei n = 0 - 6 sein kann; oder
- R5: Phenyl, 1- oder 2-Naphthyl;
- und R5: substituiert ist mit NH-SO₂-(C₁-C₆)-Alkyl, NH-SO₂-Phenyl, wobei der Phenylring bis zu zweifach mit F, Cl, CN, OH, (C₁-C₆)-Alkyl, O-(C₁-C₆)-Alkyl, CF₃, COOH, COO(C₁-C₆)-Alkyl, CONH₂ substituiert sein kann; (CH₂)n-SO₂-(C₁-C₆)-Alkyl, wobei n = 1 - 6 sein kann, (CH₂)m-SO₂-NH₂, (CH₂)m-SO₂-NH-(C₁-C₆)-Alkyl, (CH₂)m-SO₂-N((C₁-C₆)-Alkyl)₂ oder (CH₂)m-SO₂-N(=CH-N(CH₃)₂), wobei m = 0 - 6 sein kann;
- und R5: gegebenenfalls zusätzlich mit F, Cl, Br, OH, CF₃, CN, OCF₃, O-(C₁-C₆)-Alkyl, (C₁-C₆)-Alkyl, COOH, COO(C₁-C₆)Alkyl, CONH₂, NH₂, NH-CO-(C₁-C₆)-Alkyl, NH-CO-Phenyl, (CH₂)n-Phenyl, O-(CH₂)n-Phenyl, SO₂-(CH₂)n-Phenyl, wobei n = 0-3 sein kann, substituiert sein kann;
sowie deren physiologisch verträgliche Salze.

Die Erfindung bezieht sich auf Verbindungen der Formel I, in Form ihrer Racemate, racemischen Mischungen und reinen Enantiomere sowie auf ihre Diastereomere und Mischungen davon.
Die Alkyl-, Alkenyl- und Alkinylreste in den Substituenten R1, R1', R2, R3 und R5 können sowohl geradkettig wie verzweigt sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-,
Milch-, Lactobion-, Malein-, Äpfel-, Methansulfon-, Bernstein-, p-Toluolsulfon-, Wein- und Trifluoressigsäure. Für medizinische Zwecke wird in besonders bevorzugter Weise das Chlorsalz verwendet. Geeignete pharmazeutisch verträgliche basische Salze sind Ammoniumsalze, Alkalimetallsalze (wie Natriumund Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze, Solvate und physiologisch funktionellen Derivate wie hierin beschrieben.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht des vom Salz abgeleiteten Dihydrothiazolium-lons. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinalacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberfiächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wäßrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel (I) mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wäßrigen Lösung, gelöst und/oder dispergiert in einem Haftmittel oder dispergiert in einem Polymer. Eine geeignete WirkstoffKonzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Gegenstand der Erfindung ist weiterhin ein Verfahren zur Herstellung der Verbindungen der allgemeinen Formel I, dadurch gekennzeichnet, daß man die Verbindungen der Formel I so gewinnt, daß gemäß dem folgenden Reaktionsschema vorgegangen wird:

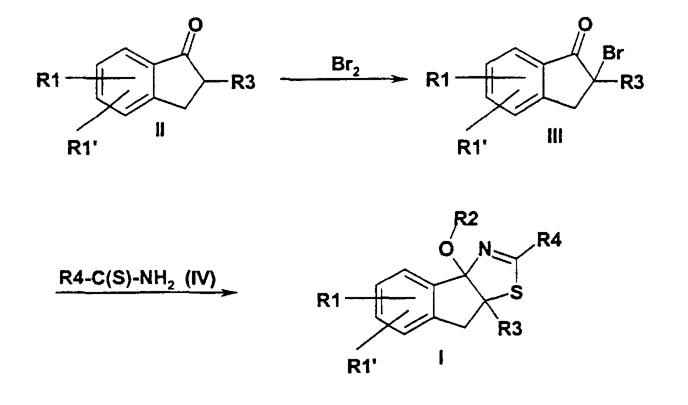

Dazu werden Verbindungen der allgemeinen Formel II, 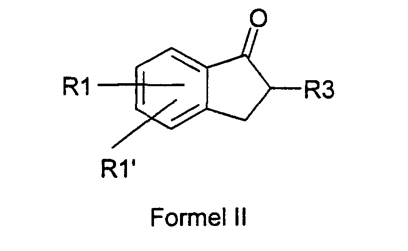 worin R1 und R1' die zu Formel I beschriebenen Bedeutungen haben, mit Brom zu einer Verbindung der Formel III, worin R3 die zu Formel I beschriebenen Bedeutungen haben kann, umgesetzt.
Verbindungen der Formel III werden sodann mit Thioamiden der Formel IV 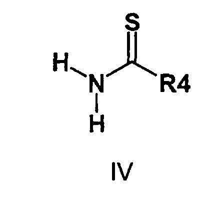 worin R4 die zu Formel I angegebene Bedeutung besitzt, zu Verbindungen der Formel I umgesetzt, worin R2 gleich Wasserstoff ist.
Diese Verbindungen können ihrerseits unter Einsatz von Standardmethoden in Verbindungen der Formel 1, worin R2 die zu Formel I beschriebenen Bedeutungen hat, umgesetzt werden.

Die Verbindungen der Formel I können auch als Salze mit Säuren vorliegen. Als anorganische Säuren kommen beispielsweise in Betracht:
Halogenwasserstoffsäuren wie Chlorwasserstoffsäure und Bromwasserstoffsäure, sowie Schwefelsäure, Phosphorsäure und Amidosulfonsäure.

Als organische Säuren seien beispielsweise genannt: Ameisensäure, Essigsäure, Benzoesäure, p-Toluolsulfonsäure, Benzolsulfonsäure, Bemsteinsäure, Fumarsäure, Maleinsäure, Milchsäure, Weinsäure, Zitronensäure, L-Ascorbinsäure, Salizylsäure, Isäthionsäure, Methansulfonsäure, Trifluormethansulfonsäure, 1,2-Benzisothiazol-3(2H)-on, 6-Methyl-1,2,3-oxathiazin-4(3H)-on-2,2-dioxid.

Die oben beschriebene Verfahrensweise wird vorteilhaft so ausgeführt, daß man die Verbindungen III mit den Thioamiden IV im molaren Verhältnis von 1:1 bis 1:1,5 umsetzt. Die Reaktion wird vorteilhaft in einem inerten Lösemittel, z.B. in polaren organischen Lösemitteln wie Dimethylformamid, Dimethylacetamid, N-Methyl-2-pyrrolidon, Dioxan, Tetrahydrofuran, Acetonitril, Nitromethan oder Diethylenglykoldimethylether durchgeführt. Als besonders vorteilhafte Lösemittel erweisen sich jedoch Essigsäuremethylester und Essigsäureethylester, kurzkettige Alkohole wie Methanol, Ethanol, Propanol, Isopropanol, sowie niedere Dialkylketone, wie z.B. Aceton, Butan-2-on oder Hexan-2-on. Auch Gemische der angeführten Reaktionsmedien können angewandt werden; so wie auch Gemische der aufgeführten Lösemittel mit Solventien, die für sich alleine genommen weniger geeignet sind, verwendet werden können, wie z.B. Gemische aus Methanol mit Benzol, Ethanol mit Toluol, Methanol mit Diethylether oder mit tert.Butylmethylether, Ethanol mit Tetrachlormethan, Aceton mit Chloroform, Dichlormethan oder 1,2-Dichlorethan, wobei das jeweils polarere Lösemittel zweckmäßigerweise im Überschuß verwendet werden soll. Die Reaktionspartner können im jeweiligen Reaktionsmedium suspendiert oder gelöst vorliegen. Grundsätzlich können die Reaktionspartner auch ohne Lösemittel umgesetzt werden, insbesondere dann, wenn das jeweilige Thioamid einen möglichst tiefen Schmelzpunkt hat. Die Reaktion verläuft nur wenig exotherm und kann zwischen - 10°C und 150°C, bevorzugt zwischen 30°C und 100°C, durchgeführt werden. Als besonders günstig erwies sich in der Regel ein Temperaturbereich zwischen 50°C und 90°C.

Die Reaktionsdauer ist weitgehend von der Reaktionstemperatur abhängig und liegt zwischen 2 Minuten und 3 Tagen bei höheren bzw. niedrigeren Temperaturen. Im günstigen Temperaturbereich liegt die Reaktionsdauer im allgemeinen zwischen 5 Minuten und 48 Stunden.

Häufig scheiden sich die Verbindungen I in Form ihrer Säureadditionssalze im Verlauf der Reaktion schwerlöslich ab, zwechmäßig wird nachträglich noch ein geeignetes Fällungsmittel zugesetzt. Als solches verwendet man z.B. Kohlenwasserstoffe wie Benzol, Toluol, Cyclohexan oder Heptan oder Tetrachlormethan; insbesondere erwiesen sich Essigsäurealkylester wie Essigsäureethylester oder Essigsäure-n-butylester oder Dialkylether wie Diethylether, Diisopropylether, Di-n-butylether oder tert.Butylmethylether als besonders geeignet. Bleibt nach Ende der Reaktion das Reaktionsgemisch in Lösung, so kann man die Salze der Verbindungen I, ggf. nach Konzentrierung der Reaktionslösung, mit einem der genannten Fällungsmittel ausfällen. Femer kann man die Lösung des Reaktionsgemisches auch vorteilhaft in die Lösung eines der genannten Fällungsmittel unter Rühren einfiltrieren. Die Aufarbeitung des Reaktionsgemisches kann auch so durchgeführt werden, daß die Reaktionsmischung unter Zusatz einer organischen Base, wie z.B. Triethylamin oder Diisobutylamin oder Ammoniak oder Morpholin oder Piperidin oder 1,8-Diazabicyclo[5.4.0]undec-7-en alkalisch gestellt wird, und das Reaktionsrohprodukt nach Konzentrierung chromatographisch, z.B. über eine Kieselgelsäule, gereinigt wird. Als geeignete Elutionsmedien dafür erweisen sich z.B. Gemische von Essigsäureethylester mit Methanol, Gemische von Dichlormethan mit Methanol, Gemische von Toluol mit Methanol oder Essigsäureethylester oder Gemische von Essigsäurethylester mit Kohlenwasserstoffen wie Heptan. Erfolgt die Reinigung des Rohproduktes auf die zuletzt beschriebene Weise, kann aus der so gewonnenen reinen Base der Formel I ein Säureadditionsprodukt der Formel I so gewonnen werden, daß man die Base in einem organischen protischen Lösemittel wie Methanol, Ethanol, Propanol oder Isopropanol oder in einem organischen aprotischen Lösemittel wie Essigsäureethylester, Diethylether, Diisiopropylether, tert.Butylmethylether, Dioxan, Tetrahydrofuran, Aceton oder Butan-2-on löst oder suspendiert und diese Mischung anschließend mit einer wenigstens äquimolaren Menge einer anorganischen Säure wie z.B. Chlorwasserstoffsäure, gelöst in einem inerten Lösemittel wie z.B. Diethylether oder Ethanol, oder einer anderen der weiter oben genannten anorganischen oder organischen Säuren versetzt.

Die Verbindungen der Formel I können aus einem inerten, geeigneten Lösemittel wie z.B. Aceton, Butan-2-on, Acetonitril, Nitromethan umkristallisiert werden. Besonders vorteilhaft ist aber die Umfällung aus einem Lösemittel wie z.B. Dimethylformamid, Dimethylacetamid, Nitromethan, Acetonitril, vorzugsweise Methanol oder Ethanol.
Die Reaktion der Verbindungen der Formel III mit den Thioamiden der Formel IV kann auch so durchgeführt werden, daß man zur Reaktionsmischung eine wenigstens äquimolare Menge einer Base, wie z.B. Triethylamin, zufügt und die so erhaltenen Verbindungen I anschließend gegebenenfalls in ihre Säureadditionsprodukte überführt.

Die Säureadditionsprodukte I können durch Behandlung mit Basen zu den Verbindungen der allgemeinen Formel I (freie Base) umgesetzt werden. Als Basen kommen beispielsweise Lösungen anorganischer Hydroxide, wie Lithium-, Natrium-, Kalium-, Calcium- oder Bariumhydroxid, Carbonate oder Hydrogencarbonate, wie Natrium- oder Kaliumcarbonat, Natrium- oder Kaliumhydrogencarbonat, Ammoniak und Amine, wie Triethylamin, Diisopropylamin, Dicyclohexylamin, Piperidin, Morpholin, Methyldicyclohexylamin in Frage.

Thioamide der allgemeinen Formel IV sind entweder kommerziell erhältlich oder können z.B. durch Umsetzung des entsprechenden Carbonsäureamids mit Phosphorpentasulfid in Pyridin (R. N. Hurd, G. Delameter, Chem. Rev. 61, 45 (1961)), oder mit Lawesson's Reagenz in Toluol, Pyridin, Hexamethylphosphorsäurtriamid [Scheibye, Pedersen und Lawesson: Bull. Soc. Chim. Belges 87, 229 (1978)], vorzugsweise in einem Gemisch von Tetrahydrofuran mit 1,3-Dimethyl-3,4,5,6-tetrahydro-2(1H)-pyrimidinon oder 1,3-Dimethyl-2-lmidazolidinon gewonnen werden. Hydroxy-, Amino- oder zusätzliche Carbonylfunktionen werden dabei zweckmäßigerweise mit einer wiederabspaltbaren Schutzfunktion, wie z. B. einem Benzyl-, tert.Butyloxycarbonyl-, Benzyloxycarbonylrest bzw. durch Überführung in ein, gegebenfalls cyclisches, Acetal überführt. Methoden dazu sind z. B. beschrieben in Th. W. Greene und P. G. M. Wuts, Protective Groups in Organic Synthesis, Second Edition, 1991, John Wiley & Sons, New York.

Thioamide der Formel IV sind auch erhältlich, indem man Nitrile der allgemeinen Formel VII

N≡C―R4 Formel VII

mit Schwefelwasserstoff (Houben-Weyl IX, 762) oder Thioacetamid (E. C. Taylor, J. A. Zoltewicz, J. Am. Chem. Soc. 82, 2656 (1960)) oder O,O-Diethyldithiophosphorsäure umsetzt. Die Umsetzungen mit Schwefelwasserstoff werden vorzugsweise in einem organischen Lösemittel wie Methanol oder Ethanol, die mit Thioacetamid in einem Lösemittel wie Dimethylformamid unter Hinzufügung von Salzsäure, die mit O,O-Diethyl-dithiophosphorsäure in einem Lösemittel wie Essigsäureethylester unter sauren, z.B. HCI, Bedingungen bei Raumtemperatur oder unter Erwärmung durchgeführt.

Die nachfolgend aufgeführten Beispiele dienen zur Erläuterung der Erfindung, ohne diese jedoch einzuschränken. Die gemessenen Fest-, bzw.

Zersetzungspunkte (Fp.) wurden nicht korrigiert und sind generell von der Aufheizgeschwindigkeit abhängig.

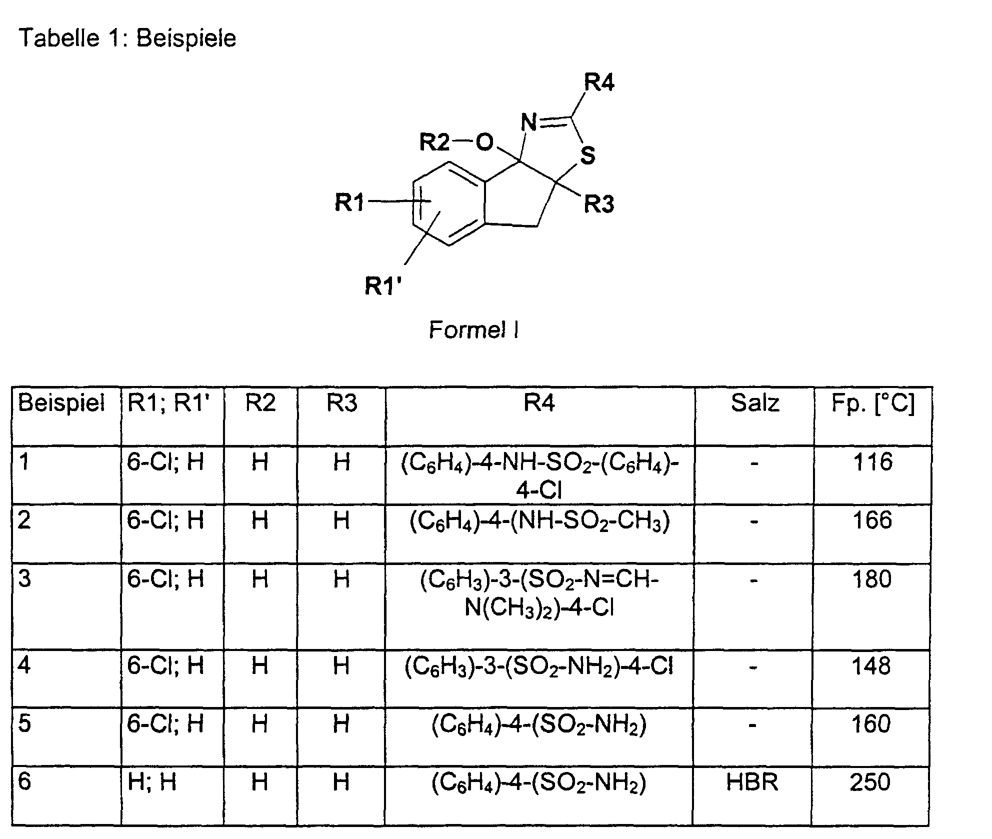

Die Verbindungen der Formel I zeichnen sich durch günstige Wirkungen auf den Fettstoffwechsel aus, insbesondere sind sie als Anorektika geeignet. Die Verbindungen können allein oder in Kombination mit weiteren anorektischen Wirkstoffen eingesetzt werden. Solche weiteren anorektischen Wirkstoffe werden z.B. in der Roten Liste, Kapitel 01 unter Abmagerungsmittel/Appetitzügler genannt. Die Verbindungen eignen sich zur Prophylaxe sowie insbesondere zur Behandlung von Obesitas. Die Verbindungen eignen sich weiterhin zur Prophylaxe sowie insbesondere zur Behandlung von Typ II Diabetes.

Die Wirksamkeit der Verbindungen wurde wie folgt getestet:

### Biologisches Prüfmodell:

Die Prüfung der anorektischen Wirkung erfolgte an männlichen oder weiblichen NMRI Mäusen. Nach 24stündigem Futterentzug wurde über eine Schlundsonde das Testpräparat verabreicht. In Einzelhaltung und bei freiem Zugang zu Trinkwasser wurde den Tieren 30 Minuten nach Präparatgabe Kondensmilch angeboten. Der Kondensmilchverbrauch wurde halbstündlich 3 Stunden lang bestimmt und das Allgemeinbefinden der Tiere beobachtet. Der gemessene Milchverbrauch wurde mit dem unbehandelter Kontrolltieren verglichen.

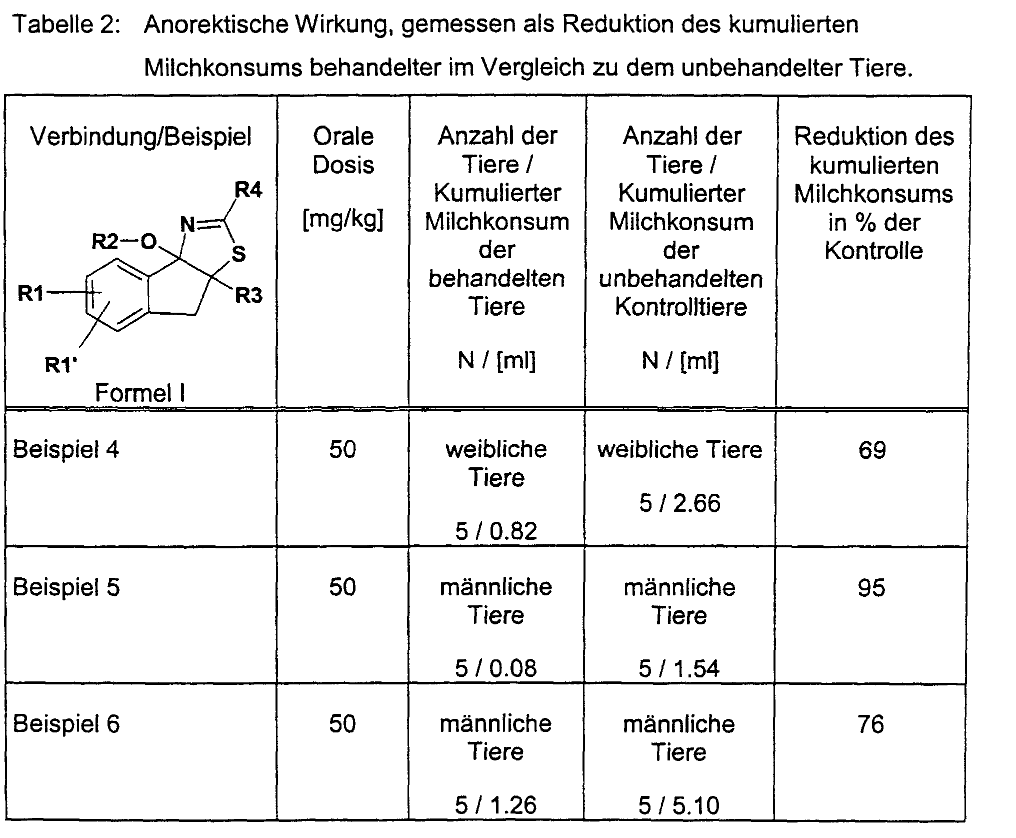

Aus der Tabelle ist abzulesen, daß die Verbindungen der Formel I eine sehr gute anorektische Wirkung zeigen.

Nachfolgend wird die Herstellung einiger Beispiele detailliert beschrieben, die übrigen Verbindungen der Formel I wurden analog erhalten:

### Beispiel 1 (Verbindung 1):

### 4-Chlor-N-[4-(6-chlor-3a-hydroxy-8,8a-dihydro-3aH-indeno[1,2-d]thiazol-2-yl)-phenyl]-benzolsulfonamid:

### a) 2-Brom-5-chlor-indan-1-on:

10 g (0.06 Mol) 5-Chlor-indan-1-on werden bei Raumtemperatur in 120 ml Eisessig unter Rühren gelöst. Man tropft 0.05 ml einer 48%igen Lösung von HBr in Wasser und anschließend 3.074 ml (0.06 mol) Brom, gelöst in 25 ml Eisessig, zu. Nach 2 h Rühren bei Raumtemperatur ist die Reaktion beendet (DC-Kontrolle).Die Lösung des Rohproduktes wird unter Rühren langsam in 300 ml Eiswasser eingetropft. Das ausgefallene Rohprodukt wird abgesaugt und gründlich mit Wasser gewaschen. Der feuchte Rückstand wird mit Essigsäureethylester vom Filter abgelöst und die Phasen des Filtrats getrennt. Die organische Phase wird über Natriumsulfat getrocknet, filtriert und im Vakuum eingeengt. Der Rückstand wird in 120 ml n-Heptan heiß gelöst; die heiße Lösung wird über ein Faltenfilter filtriert und die Lösung dann bei 0°C der Kristallisation überlassen. Das kristallisierte Produkt wird abgesaugt und im Vakuum getrocknet. Fp.: 94-96°C

### b) 4-(4-Chlor-benzolsulfonylamino)-thiobenzamid:

0.26 g 4-Chlor-4'-cyanobenzolsulfonamid werden in 10 ml absolutem Ethanol suspendiert, mit 0.15 ml Diethyldithiophosphat versetzt und 8 h unter Rückfluß gerührt. Danach gibt man eine weitere Portion 0.15 ml Diethyldithiophosphat zu und rührt weitere 12 h bei Rückflußtemperatur. Die abgekühlte Reaktionsmischung wird im Vakuum eingeengt; der Rückstand wird in Dichlormethan verrührt, der feste Rückstand abgesaugt, mit Dichlormethan gewaschen und im Vakuum getrocknet. Das so gewonnene 4-(4-Chlor-benzolsulfonylamino)-thiobenzamid wird ohne weitere Reinigung in die nächste Stufe eingesetzt.

### c) 4-Chlor-N-[4-(6-chlor-3a-hydroxy-8,8a-dihydro-3aH-indeno[1,2-d]thiazol-2-yl)- phenyl]-benzolsulfonamid:

0.15 g der Verbindung des Beispiels 1b und 0.11 g der Verbindung des Beispiels 1a werden bei Raumtemperatur in 5 ml trockenem Aceton gelöst und anschließend 4 h bei Raumtemperatur gerührt. Man fügt 65 µl Triethylamin zu und rührt über Nacht bei Raumtemperatur. Es werden weitere 20 µl Triethylamin zugefügt, und die Mischung wird eine weitere Nacht bei Raumtemperatur gerührt. Danach wird die Reaktionsmischung im Vakuum eingeengt; der Rückstand wird in Essigsäureethylester gelöst, zweimal mit Wasser und einmal mit ges. Kochsalzlösung gewaschen, über Magnesiumsulfat getrocknet, filtriert und im Vakuum eingeengt. Der Rückstand wird chromatographisch an Kieselgel mit Toluol/Aceton 3/1 gereinigt. Man erhält 4-Chlor-N-[4-(6-chlor-3a-hydroxy-8,8a-dihydro-3aHindeno[1,2-d]thiazol-2-yl)- phenyl]-benzolsulfonamid mit dem Schmelzpunkt 116°C.

### Beispiel 2 (Verbindung 6):

4-(6-Chlor-3a-hydroxy-8,8a-dihydro-3aH-indeno[1,2-d]thiazol-2-yl)- )-benzolsulfonamid wird auf analoge Weise durch Reaktion von 2-Brom-5-chlorindan-1-on mit 4-Sulfamoyl-thiobenzamid hergestellt. Die Verbindung weist einen Schmelzpunkt von 160°C auf.

## Patentansprüche

1. Verbindungen der Formel I, 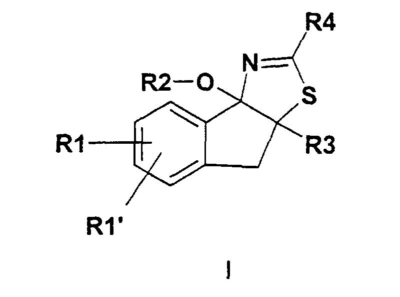 worin bedeuten
R1, R1' unabhängig voneinander H, F, Cl, Br, J, CF₃, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können, oder ein Wasserstoff durch OH, OC(O)CH₃, OC(O)H, O-CH₂-Ph, NH₂, NH-CO-CH₃ oder N(COOCH₂Ph)₂ ersetzt sein kann; SO₂-NH₂, SO₂NH(C₁-C₆)-Alkyl, SO₂N[(C₁-C₆)-Alkyl]₂, S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; NH₂, NH-(C₁-C₆)-Alkyl, N((C₁-C₆)-Alkyl)₂, NH(C₁-C₇)-Acyl, Phenyl, Biphenyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Biphenyl-, Naphthyl-, Pyridyl-, Furanyl-oder Thienylringe jeweils ein bis 3-fach substituiert sein können mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C1-C₆)-Alkyl, CONH₂; 1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;
R2 H, (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, C(O)-(C₁-C₆)-Alkyl, C(O)-(C₃-C₆)-Cycloalkyl, C(O)-(CH₂)ₙ-Phenyl, C(O)-(CH₂)ₙ-Thienyl, C(O)-(CH₂)ₙ-Pyridyl, C(O)-(CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C₁-C₆)-Alkyl substituiert sein kann;
R3 H, (C₁-C₆)-Alkyl, F, CN, N₃, O-(C₁-C₆)-Alkyl, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann; OC(O)CH₃, (C₂-C₆)-Alkinyl, (C₂-C₆)-Alkenyl, COO(C1-C6)-Alkyl, C(O)OH, C(O)NH₂, C(O)NHCH₃, C(O)N(CH₃)₂;
R4 (CH₂)n-R5, wobei n = 0 - 6 sein kann;
R5 Phenyl, Biphenyl, 1- oder 2-Naphthyl, 2-, 3- oder 4-Pyridyl, 2- oder 3-Thienyl, 2- oder 3-Furyl, 2-, 4- oder 5-Thiazolyl, 2-, 4- oder 5-Oxazolyl, 1-Pyrazolyl, 3- oder 5-Isoxazolyl, 2- oder 3-Pyrrolyl, 2- oder 3-Pyridazinyl, 2-, 4- oder 5-Pyrimidinyl, 2-Pyrazinyl, 2-(1,3,5-Triazinyl), 2- oder 5-Benzimidazolyl, 2-Benzothiazolyl, 1,2,4-Triazol-3-yl, 1,2,4-Triazol-5-yl, Tetrazol-5-yl, Indol-3-yl, Indol-5-yl oder N-Methyl-imidazol-2-, 4- oder -5-yl;
und R5 substituiert ist mit NH-SO₂-(C₁-C₆)-Alkyl, NH-SO₂-Phenyl, wobei der Phenylring bis zu zweifach mit F, Cl, CN, OH, (C₁-C₆)-Alkyl, O-(C₁-C₆)-Alkyl, CF₃, COOH, COO(C₁-C₆)-Alkyl, CONH₂ substituiert sein kann; (CH₂)n-SO₂-(C₁-C₆)-Alkyl, wobei n = 1 - 6 sein kann, (CH₂)m-SO₂-NH₂ , (CH₂)m-SO₂-NH-(C₁-C₆)-Alkyl, (CH₂)m-SO₂-N((C₁-C₆)-Alkyl)₂ oder (CH₂)m-SO₂-N(=CH-N(CH₃)₂), wobei m = 0 - 6 sein kann;
und R5 gegebenenfalls zusätzlich mit F, Cl, Br, OH, CF₃, NO₂, CN, OCF₃, O-(C₁-C₆)-Alkyl, S-(C₁-C₆)-Alkyl, SO-(C₁-C₆)-Alkyl, (C₁-C₆)-Alkyl, (C₃-C₆)-Cycloalkyl, COOH, COO(C₁-C₆)Alkyl, COO(C₃-C₆)Cycloalkyl, CONH₂, CONH(C₁-C₆)Alkyl, CON[(C₁-C₆)Alkyl]2, CONH(C₃-C₆)Cycloalkyl, NH₂, NH-CO-(C₁-C₆)-Alkyl, NH-CO-Phenyl, Pyrrolidin-1-yl, Morpholin-1-yl, Piperidin-1-yl, Piperazin-1-yl, 4-Methyl-piperazin-1-yl, (CH₂)n-Phenyl, O-(CH₂)n-Phenyl, S-(CH₂)n-Phenyl, SO₂-(CH₂)n-Phenyl, wobei n = 0-3 sein kann, substituiert sein kann;
sowie deren physiologisch verträgliche Salze und physiologisch funktionelle Derivate.

2. Verbindungen der Formel I, gemäß Anspruch 1, **dadurch gekennzeichnet, daß** darin bedeuten
R1, R1' unabhängig voneinander H, F, Cl, Br, J, CF₃, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein Wasserstoff durch OH, OC(O)CH₃, OC(O)H, O-CH₂-Ph, NH₂, NH-CO-CH₃ oder N(COOCH₂Ph)₂ ersetzt sein kann; SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; NH₂, NH-(C₁-C₆)-Alkyl, N((C₁-C₆)-Alkyl)₂, NH(C₁-C₇)-Acyl, Phenyl, Biphenyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Biphenyl-, Naphthyl-, Pyridyl-, Furanyl-oder Thienylringe jeweils ein bis 3-fach substituiert sein können mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C1-C₆)-Alkyl, CONH₂;
1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;
R2 H, (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, C(O)-(C₁-C₆)-Alkyl, C(O)-(C₃-C₆)-Cycloalkyl, C(O)-(CH₂)ₙ-Phenyl, C(O)-(CH₂)ₙ-Thienyl, C(O)-(CH₂)ₙ-Pyridyl, C(O)-(CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C₁-C₆)-Alkyl substituiert sein kann;
R3 H, (C₁-C₆)-Alkyl, F, CN, N₃, O-(C₁-C₆)-Alkyl, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH, O-(C1-C₆)-Alkyl substituiert sein kann; OC(O)CH₃, (C₂-C₆)-Alkinyl, (C₂-C₆)-Alkenyl, COO(C₁-C₆)-Alkyl, C(O)OH, C(O)NH₂, C(O)NHCH₃, C(O)N(CH₃)₂;
R4 (CH₂)n-R5, wobei n = 0 - 6 sein kann;
R5 Phenyl, Biphenyl, 1- oder 2-Naphthyl, 2-, 3- oder 4-Pyridyl oder 2-oder 3-Thienyl sein kann;
und R5 substituiert ist mit NH-SO₂-(C₁-C₆)-Alkyl, NH-SO₂-Phenyl, wobei der Phenylring bis zu zweifach mit F, Cl, CN, OH, (C₁-C₆)-Alkyl, O-(C₁-C₆)-Alkyl, CF₃, COOH, COO(C₁-C₆)-Alkyl, CONH₂ substituiert sein kann; (CH₂)n-SO₂-(C₁-C₆)-Alkyl, wobei n = 1 - 6 sein kann, (CH₂)m-SO₂-NH₂, (CH₂)m-SO₂-NH-(C₁-C₆)-Alkyl, (CH₂)m-SO₂-N((C₁-C₆)-Alkyl)₂ oder (CH₂)m-SO₂-N(=CH-N(CH₃)₂), wobei m = 0 - 6 sein kann;
und R5 gegebenenfalls zusätzlich mit F, Cl, Br, OH, CF₃, CN, OCF₃, O-(C₁-C₆)-Alkyl, (C₁-C₆)-Alkyl, (C₃-C₆)-Cycloalkyl, COOH, COO(C₁-C₆)Alkyl, COO(C₃-C₆)Cycloalkyl, CONH₂, CONH(C₁-C₆)Alkyl, CON[(C₁-C₆)Alkyl]₂, NH₂, NH-CO-(C₁-C₆)-Alkyl, NH-CO-Phenyl, (CH₂)n-Phenyl, O-(CH₂)n-Phenyl, S-(CH₂)n-Phenyl, SO₂-(CH₂)n-Phenyl, wobei n = 0-3 sein kann, substituiert sein kann;
sowie deren physiologisch verträgliche Salze und physiologisch funktionelle Derivate.

3. Verbindungen der Formel I, gemäß Anspruch 1 oder 2, **dadurch gekennzeichnet, daß** darin bedeuten
R1, R1' unabhängig voneinander H, F, Cl, Br, J, CF₃, NO₂, CN, COOH, COO(C₁-C₆)Alkyl, CONH₂, (C₁-C₆)-Alkyl, (C₂-C₆)-Alkenyl, (C₂-C₆)-Alkinyl, O-(C₁-C₆)-Alkyl, SO₂-NH₂, Phenyl, O-(CH₂)ₙ-Phenyl, wobei n = 0 - 6 sein kann, wobei die Phenylringe jeweils ein bis 3-fach substituiert sein können mit F, Cl, Br, J, OH, CF₃, NO₂, CN, OCF₃, O-(C₁-C₆)-Alkyl, (C₁-C₆)-Alkyl, NH₂, CONH₂;
R2 H, (C₁-C₆)-Alkyl, (C₃-C₆)-Cycloalkyl, (CH₂)ₙ-Phenyl, C(O)-(C₁-C₆)-Alkyl, C(O)-(CH₂)ₙ-Phenyl, wobei n = 0 - 5 sein kann und worin Phenyl bis zu zweimal mit Cl, F, CN, CF₃, (C₁-C₃)-Alkyl, OH, O-(C1-C₆)-Alkyl substituiert sein kann;
R3 H, (C₁-C₆)-Alkyl, F, (CH₂)ₙ-Phenyl, wobei n = 0 - 5 sein kann und worin Phenyl bis zu zweimal mit Cl, F, CN, CF₃, (C₁-C₃)-Alkyl, OH, O-(C₁-C₆)-Alkyl substituiert sein kann; COO(C₁-C₆)-Alkyl, C(O)OH, C(O)NH₂ ;
R4 (CH₂)n-R5, wobei n = 0 - 6 sein kann; oder
R5 Phenyl, 1- oder 2-Naphthyl;
und R5 substituiert ist mit NH-SO₂-(C₁-C₆)-Alkyl, NH-SO₂-Phenyl, wobei der Phenylring bis zu zweifach mit F, Cl, CN, OH, (C₁-C₆)-Alkyl, O-(C₁-C₆)-Alkyl, CF₃, COOH, COO(C₁-C₆)-Alkyl, CONH₂ substituiert sein kann; (CH₂)n-SO₂-(C₁-C₆)-Alkyl, wobei n = 1 - 6 sein kann, (CH₂)m-SO₂-NH₂ , (CH₂)m-SO₂-NH-(C₁-C₆)-Alkyl, (CH₂)m-SO₂-N((C₁-C₆)-Alkyl)₂ oder (CH₂)m-SO₂-N(=CH-N(CH₃)₂), wobei m = 0 - 6 sein kann;
und R5 gegebenenfalls zusätzlich mit F, Cl, Br, OH, CF₃, CN, OCF₃, O-(C₁-C₆)-Alkyl, (C₁-C₆)-Alkyl, COOH, COO(C₁-C₆)Alkyl, CONH₂, NH₂, NH-CO-(C₁-C₆)-Alkyl, NH-CO-Phenyl, (CH₂)n-Phenyl, O-(CH₂)n-Phenyl, SO₂-(CH₂)n-Phenyl, wobei n = 0-3 sein kann, substituiert sein kann;
sowie deren physiologisch verträgliche Salze.

4. Arzneimittel enthaltend eine oder mehrere der Verbindungen gemäß einem oder mehreren der Ansprüche 1 bis 3.

5. Arzneimittel enthaltend eine oder mehrere der Verbindungen gemäß einem oder mehreren der Ansprüche 1 bis 3 und ein oder mehrere anorektische Wirkstoffe.

6. Verbindungen gemäß einem oder mehreren der Ansprüche 1 bis 3 zur Anwendung als Medikament zur Prophylaxe oder Behandlung der Obesitas.

7. Verbindungen gemäß einem oder mehreren der Ansprüche 1 bis 3 zur Anwendung als Medikament zur Prophylaxe oder Behandlung des Typ II Diabetes.

8. Verbindungen gemäß einem oder mehreren der Ansprüche 1 bis 3 in Kombination mit mindestens einem weiteren anorektischen Wirkstoff zur Anwendung als Medikament zur Prophylaxe oder Behandlung der Obesitas.

9. Verbindungen gemäß einem oder mehreren der Ansprüche 1 bis 3 in Kombination mit mindestens einem weiteren anorektischen Wirkstoff zur Anwendung als Medikament zur Prophylaxe oder Behandlung der des Typ II Diabetes.

10. Verfahren zur Herstellung eines Arzneimittels enthaltend eine oder mehrere der Verbindungen gemäß einem oder mehreren der Ansprüche 1 bis 3, **dadurch gekennzeichnet, daß** der Wirkstoff mit einem pharmazeutisch geeigneten Träger vermischt wird und diese Mischung in eine für die Verabreichung geeignete Form gebracht wird.

11. Verwendung der Verbindungen gemäß einem oder mehreren der Ansprüche 1 bis 3 zur Herstellung eines Medikaments zur Prophylaxe oder Behandlung der Obesitas.

12. Verwendung der Verbindungen gemäß einem oder mehreren der Ansprüche 1 bis 3 zur Herstellung eines Medikaments zur Prophylaxe oder Behandlung des Typ II Diabetes.

13. Verfahren zur Herstellung der Verbindungen gemäß einem oder mehreren der Ansprüche 1 bis 3, **dadurch gekennzeichnet, daß** nach folgendem Formelschema 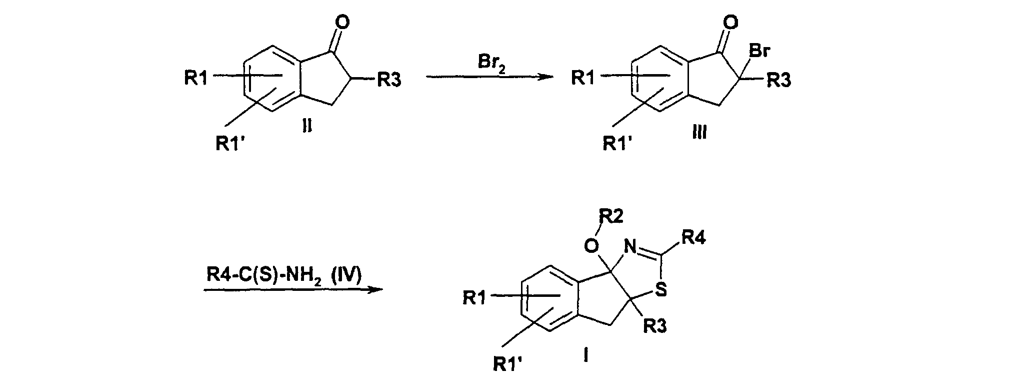 eine Verbindung der Formel II, worin R1 und R1' und R3 die zu Formel I angegebenen Bedeutungen haben, mit Brom zu einer Verbindung III worin R1, R1' und R3 die zu Formel I angegebene Bedeutung haben, umgesetzt wird, und daß die Verbindung der Formel III weiter mit Thioamiden der Formel IV, worin R4 die zu Formel I angegebene Bedeutung hat, zu einer Verbindung der Formel I umgesetzt wird.

## Claims

1. A compound of the formula I 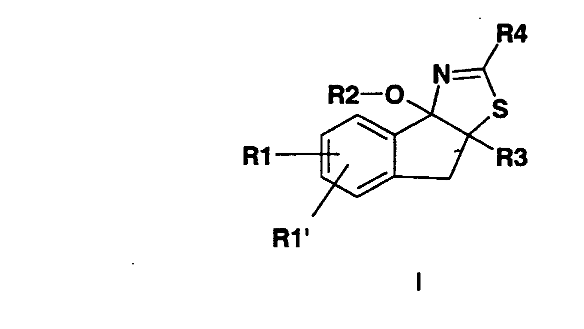 in which
R1, R1' independently of one another are H, F, Cl, Br, I, CF₃, NO₂, CN, COOH, COO(C₁-C₆)-alkyl, CONH₂, CONH(C₁-C₆)-alkyl, CON[(C₁-C₆)-alkyl]₂, (C₁-C₆)-alkyl, (C₂-C₆)-alkenyl, (C₂-C₆)-alkynyl, O-(C₁-C₆)-alkyl, where in the alkyl radicals one or more, or all the hydrogen(s) may be replaced by fluorine, or one hydrogen may be replaced by OH, OC(O)CH₃, OC(O)H, O-CH₂-Ph, NH₂, NH-CO-CH₃ or N(COOCH₂Ph)₂;
are SO₂-NH₂, SO₂NH(C₁-C₆)-alkyl, SO₂N[(C₁-C₆)-alkyl]₂, S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n can be 0-6 and the phenyl radical can be substituted up to two times by F, Cl, Br, OH, CF₃, NO₂, CN, OCF₃, O-(C₁-C₆)-alkyl, (C₁-C₆)-alkyl, NH₂;
are NH₂, NH-(C₁-C₆)-alkyl, N[(C₁-C₆)-alkyl]₂, NH(C₁-C₇)-acyl, phenyl, biphenyl, O-(CH₂)ₙ-phenyl, where n can be 0-6, 1- or 2-naphthyl, 2-, 3- or 4-pyridyl, 2- or 3-furanyl or 2- or 3-thienyl, where the phenyl, biphenyl, naphthyl, pyridyl, furanyl or thienyl rings can in each case be substituted one to three times by F, Cl, Br, I, OH, CF₃, NO₂, CN, OCF₃, O-(C₁-C₆)-alkyl, (C₁-C₆)-alkyl, NH₂, NH(C₁-C₆)-alkyl, N[(C₁-C₆)-alkyl]₂, SO₂-CH₃, COOH, COO-(C₁-C₆)-alkyl, CONH₂;
are 1,2,3-triazol-5-yl, where the triazole ring can be substituted in the 1-, 2- or 3-position by methyl or benzyl; are tetrazol-5-yl, where the tetrazole ring can be substituted in the 1-or 2-position by methyl or benzyl;
R2 is H, (C₁-C₆)-alkyl, (C₃-C₆)-cycloalkyl, (CH₂)ₙ-phenyl, (CH₂)ₙ-thienyl, (CH₂)ₙ-pyridyl, (CH₂)ₙ-furyl, C(O)-(C₁-C₆)-alkyl, C(O)-(C₃-C₆)-cycloalkyl, C(O)-(CH₂)ₙ-phenyl, C(O)-(CH₂)ₙ-thienyl, C(O)-(CH₂)ₙ-pyridyl, C(O)-(CH₂)ₙ-furyl, where n can be 0-5 and in which phenyl, thienyl, pyridyl and furyl can in each case be substituted up to two times by Cl, F, CN, CF₃, (C₁-C₃)-alkyl, OH, O-(C₁-C₆)-alkyl;
R3 is H, (C₁-C₆)-alkyl, F, CN, N₃, O-(C₁-C₆)-alkyl, (CH₂)ₙ-phenyl, (CH₂)ₙ-thienyl, (CH₂)ₙ-pyridyl, (CH₂)ₙ-furyl, where n can be 0-5 and in which phenyl, thienyl, pyridyl and furyl can in each case be substituted up to two times by Cl, F, CN, CF₃, (C₁-C₃)-alkyl, OH, O-(C₁-C₆)-alkyl;
is OC(O)CH₃, (C₂-C₆)-alkynyl, (C₂-C₆)-alkenyl, COO(C₁-C₆)-alkyl, C(O)OH, C(O)NH₂, C(O)NHCH₃, C(O)N(CH₃)₂;
R4 is (CH₂)ₙ-R5, where n can be 0-6;
R5 is phenyl, biphenyl, 1- or 2-naphthyl, 2-, 3- or 4-pyridyl, 2- or 3-thienyl, 2- or 3-furyl, 2-, 4- or 5-thiazolyl, 2-, 4- or 5-oxazolyl, 1-pyrazolyl, 3- or 5-isoxazolyl, 2- or 3-pyrrolyl, 2- or 3-pyridazinyl, 2-, 4- or 5-pyrimidinyl, 2-pyrazinyl, 2-(1,3,5-triazinyl), 2- or 5-benzimidazolyl, 2-benzothiazolyl, 1,2,4-triazol-3-yl, 1,2,4-triazol-5-yl, tetrazol-5-yl, indol-3-yl, indol-5-yl or N-methylimidazol-2-, -4- or -5-yl;
and R5 is substituted by NH-SO₂-(C₁-C₆)-alkyl, NH-SO₂-phenyl, where the phenyl ring can be substituted up to two times by F, Cl, CN, OH, (C₁-C₆)-alkyl, O-(C₁-C₆)-alkyl, CF₃, COOH, COO(C₁-C₆)-alkyl, CONH₂; (CH₂)ₙ-SO₂-(C₁-C₆)-alkyl, where n can be 1-6, (CH₂)ₘ-SO₂-NH₂ , (CH₂)ₘ-SO₂-NH-(C₁-C₆)-alkyl, (CH₂)ₘ-SO₂-N[(C₁-C₆)-alkyl]₂ or (CH₂)ₘ-SO₂-N(=CH-N(CH₃)₂), where m can be 0-6;
and R5 can optionally additionally be substituted by F, Cl, Br, OH, CF₃, NO₂, CN, OCF₃, O-(C₁-C₆)-alkyl, S-(C₁-C₆)-alkyl, SO-(C₁-C₆)-alkyl, (C₁-C₆)-alkyl, (C₃-C₆)-cycloalkyl, COOH, COO(C₁-C₆)-alkyl, COO(C₃-C₆)-cycloalkyl, CONH₂, CONH(C₁-C₆)-alkyl, CON[(C₁-C₆)-alkyl]₂, CONH(C₃-C₆)-cycloalkyl, NH₂, NH-CO-(C₁-C₆)-alkyl, NH-CO-phenyl, pyrrolidin-1-yl, morpholin-1-yl, piperidin-1-yl, piperazin-1-yl, 4-methylpiperazin-1-yl, (CH₂)ₙ-phenyl, O-(CH₂)ₙ-phenyl, S-(CH₂)ₙ-phenyl, SO₂-(CH₂)ₙ-phenyl, where n can be 0-3;
and its physiologically acceptable salts and physiologically functional derivatives.

2. A compound of the formula I as claimed in claim 1, wherein
R1, R1' independently of one another are H, F, Cl, Br, I, CF₃, NO₂, CN, COOH, COO(C₁-C₆)-alkyl, CONH₂, CONH(C₁-C₆)-alkyl, CON[(C₁-C₆)-alkyl]₂, (C₁-C₆)-alkyl, (C₂-C₆)-alkenyl, (C₂-C₆)-alkynyl, O-(C₁-C₆)-alkyl, where in the alkyl radicals one hydrogen may be replaced by OH, OC(O)CH₃, OC(O)H, O-CH₂-Ph, NH₂, NH-CO-CH₃ or N(COOCH₂Ph)₂;
is SO₂-NH₂, SO₂NH(C₁-C₆)-alkyl, SO₂N[(C₁-C₆)-alkyl]₂, S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n can be 0-6 and the phenyl radical can be substituted up to two times by F, Cl, Br, OH, CF₃, NO₂, CN, OCF₃, O-(C₁-C₆)-alkyl, (C₁-C₆)-alkyl, NH₂;
is NH₂, NH-(C₁-C₆)-alkyl, N[(C₁-C₆)-alkyl]₂, NH(C₁-C₇)-acyl, phenyl, biphenyl, O-(CH₂)ₙ-phenyl, where n can be 0-6, 1- or 2-naphthyl, 2-, 3- or 4-pyridyl, 2- or 3-furanyl or 2- or 3-thienyl where the phenyl, biphenyl, naphthyl, pyridyl, furanyl or thienyl rings can in each case be substituted one to 3 times by F, Cl, Br, I, OH, CF₃, NO₂, CN, OCF₃, O-(C₁-C₆)-alkyl, (C₁-C₆)-alkyl, NH₂, NH(C₁-C₆)-alkyl, N[(C₁-C₆)-alkyl]₂, SO₂-CH₃, COOH, COO-(C₁-C₆)-alkyl, CONH₂; are 1,2,3-triazol-5-yl, where the triazole ring can be substituted in the 1-, 2- or 3-position by methyl or benzyl;
are tetrazol-5-yl, where the tetrazole ring can be substituted in the 1-or 2-position by methyl or benzyl;
R2 is H, (C₁-C₆)-alkyl, (C₃-C₆)-cycloalkyl, (CH₂)ₙ-phenyl, (CH₂)ₙ-thienyl, (CH₂)ₙ-pyridyl, (CH₂)ₙ-furyl, C(O)-(C₁-C₆)-alkyl, C(O)-(C₃-C₆)-cycloalkyl, C(O)-(CH₂)ₙ-phenyl, C(O)-(CH₂)ₙ-thienyl, C(O)-(CH₂)ₙ-pyridyl, C(O)-(CH₂)ₙ-furyl, where n can be 0-5 and in which phenyl, thienyl, pyridyl and furyl can in each case be substituted up to two times by Cl, F, CN, CF₃, (C₁-C₃)-alkyl, OH, O-(C₁-C₆)-alkyl;
R3 is H, (C₁-C₆)-alkyl, F, CN, N₃, O-(C₁-C₆)-alkyl, (CH₂)ₙ-phenyl, (CH₂)ₙ-thienyl, (CH₂)ₙ-pyridyl, (CH₂)ₙ-furyl, where n can be 0-5 and in which phenyl, thienyl, pyridyl and furyl can in each case be substituted up to two times by Cl, F, CN, CF₃, (C₁-C₃)-alkyl, OH, O-(C₁-C₆)-alkyl;
is OC(O)CH₃, (C₂-C₆)-alkynyl, (C₂-C₆)-alkenyl, COO(C₁-C₆)-alkyl, C(O)OH, C(O)NH₂, C(O)NHCH₃, C(O)N(CH₃)₂;
R4 is (CH₂)ₙ-R5, where n can be 0-6;
R5 is phenyl, biphenyl, 1- or 2-naphthyl, 2-, 3- or 4-pyridyl or 2- or 3-thienyl;
and R5 is substituted by NH-SO₂-(C₁-C₆)-alkyl, NH-SO₂-phenyl, where the phenyl ring can be substituted up to two times by F, Cl, CN, OH, (C₁-C₆)-alkyl, O-(C₁-C₆)-alkyl, CF₃, COOH, COO(C₁-C₆)-alkyl, CONH₂; (CH₂)ₙ-SO₂-(C₁-C₆)-alkyl, where n can be 1-6, (CH₂)ₘ-SO₂-NH₂ , (CH₂)ₘ-SO₂-NH-(C₁-C₆)-alkyl, (CH₂)ₘ-SO₂-N[(C₁-C₆]-alkyl)₂ or (CH₂)ₘ-SO₂-N(=CH-N(CH₃)₂), where m can be 0-6;
and R5 can optionally additionally be substituted by F, Cl, Br, OH, CF₃, CN, OCF₃, O-(C₁-C₆)-alkyl, (C₁-C₆)-alkyl, (C₃-C₆)-cycloalkyl, COOH, COO(C₁-C₆)-alkyl, COO(C₃-C₆)-cycloalkyl, CONH₂, CONH(C₁-C₆)-alkyl, CON[(C₁-C₆)-alkyl]₂, NH₂, NH-CO-(C₁-C₆)-alkyl, NH-CO-phenyl, (CH₂)ₙ-phenyl, O-(CH₂)ₙ-phenyl, S-(CH₂)ₙ-phenyl, SO₂-(CH₂)ₙ-phenyl, where n can be 0-3;
and its physiologically acceptable salts and physiologically functional derivatives.

3. A compound of the formula I as claimed in claim 1 or 2, wherein
R1, R1' independently of one another are H, F, Cl, Br, I, CF₃, NO₂, CN, COOH, COO(C₁-C₆)-alkyl, CONH₂, (C₁-C₆)-alkyl, (C₂-C₆)-alkenyl, (C₂-C₆)-alkynyl, O-(C₁-C₆)-alkyl, SO₂-NH₂, phenyl, O-(CH₂)ₙ-phenyl, where n can be 0-6, where the phenyl rings can in each case be substituted one to 3 times by F, Cl, Br, I, OH, CF₃, NO₂, CN, OCF₃, O-(C₁-C₆)-alkyl, (C₁-C₆)-alkyl, NH₂, CONH₂;
R2 is H, (C₁-C₆)-alkyl, (C₃-C₆)-cycloalkyl, (CH₂)ₙ-phenyl, C(O)-(C₁-C₆)-alkyl, C(O)-(CH₂)ₙ-phenyl, where n can be 0-5 and in which phenyl can be substituted up to two times by Cl, F, CN, CF₃, (C₁-C₃)-alkyl, OH, O-(C₁-C₆)-alkyl;
R3 is H, (C₁-C₆)-alkyl, F, (CH₂)ₙ-phenyl, where n can be 0-5 and in which phenyl can be substituted up to two times by Cl, F, CN, CF₃, (C₁-C₃)-alkyl, OH, O-(C₁-C₆)-alkyl; is COO(C₁-C₆)-alkyl, C(O)OH, C(O)NH₂;
R4 is (CH₂)ₙ-R5, where n can be 0-6; or
R5 is phenyl, 1- or 2-naphthyl;
and R5 is substituted by NH-SO₂-(C₁-C₆)-alkyl, NH-SO₂-phenyl, where the phenyl ring can be substituted up to two times by F, Cl, CN, OH, (C₁-C₆)-alkyl, O-(C₁-C₆)-alkyl, CF₃, COOH, COO(C₁-C₆)-alkyl, CONH₂; is (CH₂)ₙ-SO₂-(C₁-C₆)-alkyl, where n can be 1-6,
is (CH₂)ₘ-SO₂-NH₂ , (CH₂)ₘ-SO₂-NH-(C₁-C₆)-alkyl, (CH₂)ₘ-SO₂-N[(C₁-C₆)-alkyl]₂ or (CH₂)ₘ-SO₂-N(=CH-N(CH₃)₂), where m can be 0-6;
and R5 can optionally additionally be substituted by F, Cl, Br, OH, CF₃, CN, OCF₃, O-(C₁-C₆)-alkyl, (C₁-C₆)-alkyl, COOH, COO(C₁-C₆)-alkyl, CONH₂, NH₂, NH-CO-(C₁-C₆)-alkyl, NH-CO-phenyl, (CH₂)ₙ-phenyl, O-(CH₂)ₙ-phenyl, SO₂-(CH₂)ₙ-phenyl, where n can be 0-3;
and its physiologically acceptable salts.

4. A medicament, comprising one or more of the compounds as claimed in one or more of claims 1 to 3.

5. A medicament, comprising one or more of the compounds as claimed in one or more of claims 1 to 3 and one or more anorectics.

6. A compound as claimed in one or more of claims 1 to 3 for use as a medicament for the prophylaxis or treatment of obesity.

7. A compound as claimed in one or more of claims 1 to 3 for use as a medicament for the prophylaxis or treatment of type II diabetes.

8. A compound as claimed in one or more of claims 1 to 3 in combination with at least one further anorectic for use as a medicament for the prophylaxis or treatment of obesity.

9. A compound as claimed in one or more of claims 1 to 3 in combination with at least one further anorectic for use as a medicament for the prophylaxis or treatment of type II diabetes.

10. A process for preparing a medicament comprising one or more of the compounds as claimed in one or more of claims 1 to 3, which comprises mixing the active compound with a pharmaceutically suitable excipient and bringing this mixture into a form suitable for administration.

11. The use of a compound as claimed in one or more of claims 1 to 3 for preparing a medicament for the prophylaxis or treatment of obesity.

12. The use of a compound as claimed in one or more of claims 1 to 3 for preparing a medicament for the propphylaxis or treatment of type II diabetes.

13. A process for preparing a compound as claimed in one or more of claims 1 to 3, which comprises reacting, according to the following reaction scheme, 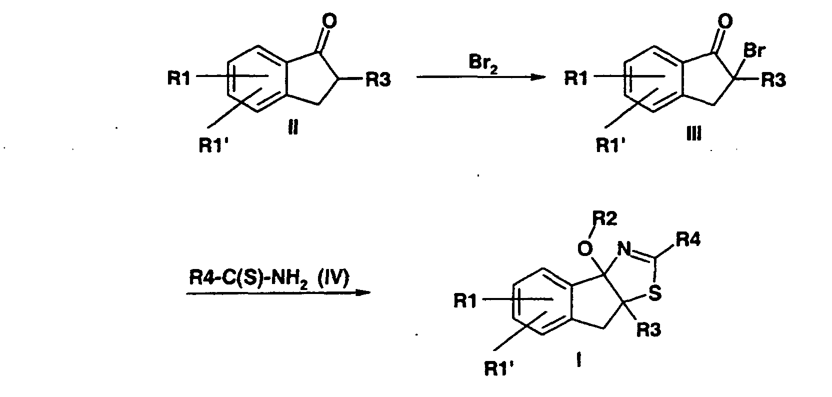 a compound of the formula II in which R1 and R1' and R3 are as defined for formula I with bromine to give a compound III in which R1, R1' and R3 are as defined for formula I, and reacting the compound of the formula III further with thioamides of the formula IV in which R4 is as defined for formula I to give a compound of the formula I.

## Revendications

1. Composés de formule I, 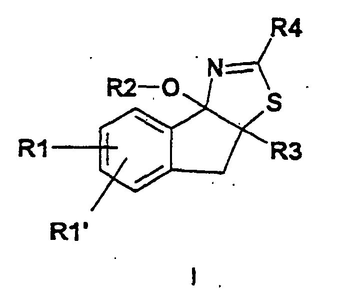 où
R1, R1' représentent indépendamment l'un de l'autre des atomes de H, F, Cl, Br, I, des groupes CF₃, NO₂, CN, COOH, COO-alkyle en C₁-C₆, CONH₂, CONH-alkyle en C₁-C₆, CON(alkyle en C₁-C₆)₂, alkyle en C₁-C₆, alcényle en C₂-C₆, alcynyle en C₂-C₆, O-alkyle en C₁-C₆, où un, plusieurs ou tous les atomes d'hydrogène peuvent être remplacés par des atomes de fluor, ou un atome d'hydrogène peut être remplacé par un groupe OH, OC(O)CH₃, OC(O)H, O-CH₂-Ph, NH₂, NH-CO-CH₃ ou N(COOCH₂Ph)₂ ; SO₂-NH₂, SO₂NH-alkyle en C₁-C₆, SO₂N(alkyle en C₁-C₆)₂, S-alkyle en C₁-C₆, S-(CH₂)ₙ-phényle, SO-alkyle en C₁-C₆, SO-(CH₂)ₙ-phényle, SO₂-alkyle en C₁-C₆, SO₂-(CH₂)ₙ-phényle, n peut être n = 0-6 et le reste phényle peut être substitué jusqu'à 2 fois par des substituants F, Cl, Br, OH, CF₃, NO₂, CN, OCF₃, O-alkyle en C₁-C₆, alkyle en C₁-C₆, NH₂ ;
NH₂, NH-alkyle en C₁-C₆, N(alkyle en C₁-C₆)₂, NH-acyle en C₁-C₇, phényle, biphényle, O-(CH₂)ₙ-phényle, n peut être n = 0-6, 1- ou 2-naphtyle, 2-, 3- ou 4-pyridyle, 2- ou 3-furanyle ou 2- ou 3-thiényle, les cycles phényle, biphényle, naphtyle, pyridyle, furanyle ou thiényle peuvent chacun être substitués une à trois fois par un substituant F, Cl, Br, I, OH, CF₃, NO₂, CN, OCF₃, O-alkyle en C₁-C₆, alkyle en C₁-C₆, NH₂, NH-alkyle en C₁-C₆, N-(alkyle en C₁-C₆)₂, SO₂-CH₃, COOH, COO-alkyle en C₁-C₆, CONH₂ ;
1,2,3-triazol-5-yle, le cycle triazole pouvant être substitué en position 1, 2 ou 3 par des substituants méthyle ou benzyle ;
tétrazol-5-yle, le cycle tétrazole pouvant être substitué en position 1 ou 2 par des substituants méthyle ou benzyle ;
R2 représente un atome de H, des groupes alkyle en C₁-C₆, cycloalkyle en C₃-C₆, (CH₂)ₙ-phényle, (CH₂)ₙ-thiényle, (CH₂)ₙ-pyridyle, (CH₂)ₙ-furyle, C(O)-alkyle en C₁-C₆, C(O)-cycloalkyle en C₃-C₆, C (O)-(CH₂)ₙ-phényle, C (O)-(CH₂)ₙ-thiényle, C(O)-(CH₂)ₙ-pyridyle, C (O)-(CH₂)ₙ-furyle, n peut être n = 0-5 et où les cycles phényle, thiényle, pyridyle, furyle peuvent chacun être substitué une à deux fois par des substituants Cl, F, CN, CF₃, alkyle en C₁-C₃, OH, O-alkyle en C₁-C₆ ;
R3 représente un atome de H, des groupes alkyle en C₁-C₆, F, CN, N₃, O-alkyle en C₁-C₆, (CH₂)ₙ-phényle, (CH₂)ₙ-thiényle, (CH₂)ₙ-pyridyle, (CH₂)ₙ-furyle, n pouvant être n = 0-5 et où les cycles phényle, thiényle, pyridyle, furyle peuvent chacun être substitué une à deux fois par des substituants Cl, F, CN, CF₃, alkyle en C₁-C₃, OH, O-alkyle en C₁-C₆ ; OC(O)CH₃, alcynyle en C₂-C₆, alcényle en C₂-C₆, COO-alkyle en C₁-C₆, C(O)OH, C(O)NH₂, C(O)NHCH₃, C(O)N(CH₃)₂ ;
R4 représente un groupe (CH₂)ₙ-R5, n peut être n = 0--6 ;
R5 représente des groupes phényle, biphényle, 1- ou 2-naphtyle, 2-, 3- ou 4-pyridyle, 2- ou 3-thiényle, 2- ou 3-furyle, 2-, 4- ou 5-thoiazolyle, 2-, 4- ou 5-oxazolyle, 1-pyrazolyle, 3- ou 5-isoxazolyle, 2- ou 3-pyrrolyle, 2- ou 3-pyridazinyle, 2-, 4- ou 5-pyrimidinyle, 2-pyrazinyle, 2-(1,3,5-triazinyle), 2- ou 5-benzimidazolyle, 2-benzothiazolyle, 1,2,4-triazol-3-yle, 1,2,4-triazol-5-yle, tétrazol-5-yle, indol-3-yle, indol-5-yle ou N-méthyl-imidazol-2-, 4- ou 5-yle ;
et R5 est substitué par des substituants NH-SO₂-alkyle en C₁-C₆, NH-SO₂-phényle, le cycle phényle pouvant être substitué jusqu'à 2 fois par des substituants F, Cl, CN, OH, alkyle en C₁-C₆, O-alkyle en C₁-C₆, CF₃, COOH, COO-alkyle en C₁-C₆, CONH₂ ;
(CH₂)ₙ-SO₂-alkyle en C₁-C₆, n peut être n = 1-6, (CH₂)ₘ-SO₂-NH₂, (CH₂)ₘ-SO₂-NH-alkyle en C₁-C₆, (CH₂)ₘ-SO₂-N-(alkyle en C₁-C₆)₂ ou (CH₂)ₘ-SO₂-N(=CH-N(CH₃)₂) m peut être m = 0-6 ;
et R5 peut éventuellement être substitué de plus par des substituants F, Cl, Br, OH, CF₃, NO₂, CN, OCF₃, O-alkyle en C₁-C₆, S-alkyle en C₁-C₆, SO-alkyle en C₁-C₆, alkyle en C₁-C₆, cycloalkyle en C₃-C₆, COOH, COO-alkyle en C₁-C₆, COO-cycloalkyle en C₃-C₆, CONH₂, CONH-alkyle en C₁-C₆, CON(alkyle en C₁-C₆)₂, CONH-cycloalkyle en C₃-C₆, NH₂, NH-CO-alkyle en C₁-C₆, NH-CO-phényle, pyrrolidin-1-yle, morpholin-1-yle, pipéridin-1-yle, pipérazin-1-yle, 4-méthyl-pipérazin-1-yle, (CH₂)ₙ-phényle, O- (CH₂)ₙ-phényle, S- (CH₂)ₙ-phényle, SO₂-(CH₂)ₙ-phényle, n peut être n = 0-3 ;
ainsi que leurs sels physiologiquement compatibles et leurs dérivés physiologiquement fonctionnels.

2. Composés de formule I selon la revendication 1, **caractérisés en ce que**
R1, R1' représentent indépendamment l'un de l'autre des atomes de H, F, Cl, Br, I, des groupes CF₃, NO₂, CN, COOH, COO-alkyle en C₁-C₆, CONH₂, CONH-alkyle en C₁-C₆, CON(alkyle en C₁-C₆)₂, alkyle en C₁-C₆, alcényle en C₂-C₆, alcynyle en C₂-C₆, O-alkyle en C₁-C₆, dans les restes alkyle, un atome d'hydrogène peut être remplacé par des groupes OH, OC(O)CH₃, OC(O)H, O-CH₂-Ph, NH₂, NH-CO-CH₃ ou N(COOCH₂Ph)₂ ;
SO₂-NH₂, SO₂NH-alkyle en C₁-C₆, SO₂N-(alkyle en C₁-C₆)₂, S-alkyle en C₁-C₆, S-(CH₂)ₙ-phényle, SO-alkyle en C₁-C₆, SO-(CH₂)ₙ-phényle, SO₂-alkyle en C₁-C₆, SO₂-(CH₂)ₙ-phényle, n peut être n = 0-6 et le reste phényle peut être substitué jusqu'à 2 fois par des substituants F, Cl, Br, OH, CF₃, NO₂, CN, OCF₃, O-alkyle en C₁-C₆, alkyle en C₁-C₆, NH₂ ;
NH₂, NH-alkyle en C₁-C₆, N-(alkyle en C₁-C₆)₂, NH-acyle en C₁-C₇, phényle, biphényle, O-(CH₂)ₙ-phényle, n peut être n = 0-6, 1- ou 2-naphtyle, 2-. 3- ou 4-pyridyle, 2- ou 3-furanyle ou 2- ou 3-thiényle, les cycles phényle, biphényle, naphtyle, pyridyle, furanyle ou thiényle peuvent chacun être substitués une à trois fois par un substituant F, Cl, Br, I, OH, CF₃, NO₂, CN, OCF₃, O-alkyle en C₁-C₆, alkyle en C₁-C₆, NH₂, NH-alkyle en C₁-C₆, N-(alkyle en C₁-C₆)₂, SO₂-CH₃, COOH, COO-alkyle en C₁-C₆, CONH₂ ;
1,2,3-triazol-5-yle, le cycle triazole pouvant être substitué en position 1, 2 ou 3 par des substituants méthyle ou benzyle ; tétrazol-5-yle, le cycle tétrazole pouvant être substitué en position 1 ou 2 par des substituants méthyle ou benzyle ;
R2 représente un atome de H, des groupes alkyle en C₁-C₆, cycloalkyle en C₃-C₆, (CH₂)ₙ-phényle, (CH₂)ₙ-thiényle, (CH₂)ₙ-pyridyle, (CH₂)ₙ-furyle, C(O)-alkyle en C₁-C₆, C(O)-cycloalkyle en C₃-C₆, C(O)-(CH₂)ₙ-phényle, C(O)-(CH₂)ₙ-thiényle, C(O)-(CH₂)ₙ-pyridyle, C(O)-(CH₂)ₙ-furyle, n peut être n = 0-5 et où les cycles phényle, thiényle, pyridyle, furyle peuvent chacun être substitué une à deux fois par des substituants Cl, F, CN, CF₃, alkyle en C₁-C₃, OH, O-alkyle en C₁-C₆ ;
R3 représente un atome de H, des groupes alkyle en C₁-C₆, F, CN, N₃, O-alkyle en C₁-C₆, (CH₂)ₙ-phényle, (CH₂)ₙ-thiényle, (CH₂)ₙ-pyridyle, (CH₂)ₙ-furyle, n pouvant être n = 0-5 et où les cycles phényle, thiényle, pyridyle, furyle, peuvent chacun être substitué une à deux fois par des substituants Cl, F, CN, CF₃, alkyle en C₁-C₃, OH, O-alkyle en C₁-C₆ ; OC(O)CH₃, alcynyle en C₂-C₆, alcényle en C₂-C₆, COO-alkyle en C₁-C₆, C(O)OH, C(O)NH₂, C(O)NHCH₃, C(O)N(CH₃)₂ ;
R4 représente un groupe (CH₂)ₙ-R5, n pouvant être n = 0-6 ;
R5 peut représenter des groupes phényle, biphényle, 1- ou 2-naphtyle, 2-, 3- ou 4-pyridyle, 2- ou 3-thiényle ;
et R5 est substitué par des substituants NH-SO₂-alkyle en C₁-C₆, NH-SO₂-phényle, le cycle phényle peut être substitué jusqu'à 2 fois par des substituants F, Cl, CN, OH, alkyle en C₁-C₆, O-alkyle en C₁-C₆, CF₃, COOH, COO-alkyle en C₁-C₆, CONH₂ ;
(CH₂)ₙ-SO₂-alkyle en C₁-C₆, n pouvant être n = 1-6,
(CH₂)ₘ-SO₂-NH₂, (CH₂)ₘ-SO₂-NH-alkyle en C₁-C₆, (CH₂)m-SO₂-N-(alkyle en C₁-C₆)₂ ou (CH₂)ₘ-SO₂-N(=CH-N(CH₃)₂, m peut être m = 0-6 ;
et R5 peut éventuellement être substitué de plus par des substituants F, Cl, Br, OH, CF₃, CN, OCF₃, O-alkyle en C₁-C₆, alkyle en C₁-C₆, cycloalkyle en C₃-C₆, COOH, COO-alkyle en C₁-C₆, COO-cycloalkyle en C₃-C₆, CONH₂, CONH-alkyle en C₁-C₆, CON(alkyle en C₁-C₆)₂, NH₂, NH-CO-alkyle en C₁-C₆, NH-CO-phényle, (CH₂)ₙ-phényle, O-(CH₂)ₙ-phényle, S-(CH₂)ₙ-phényle, SO₂-(CH₂)ₙ-phényle, n peut être n = 0-3 ;
ainsi que leurs sels physiologiquement compatibles et leurs dérivés physiologiquement fonctionnels.

3. Composés de formule I selon la revendication 1 ou 2, **caractérisés en ce que**
R1, R1' représentent indépendamment l'un de l'autre des atomes de H, F, Cl, Br, I, des groupes CF₃, NO₂, CN, COOH, COO-alkyle en C₁-C₆, CONH₂, alkyle en C₁-C₆, alcényle en C₂-C₆, alcynyle en C₂-C₆, O-alkyle en C₁-C₆, SO₂-NH₂, phényle, O-(CH₂)ₙ-phényle, n peut être n = 0-6, les cycles phényle peuvent chacun être substitué une à trois fois par des substituants F, Cl, Br, I, OH, CF₃, NO₂, CN, OCF₃, O-alkyle en C₁-C₆, alkyle en C₁-C₆, NH₂, CONH₂ ;
R2 représente un atome de H, des groupes alkyle en C₁-C₆, cycloalkyle en C₃-C₆, (CH₂)ₙ-phényle, C(O)-alkyle en C₁-C₆, C(O)-(CH₂)ₙ-phényle, n peut être n = 0-5 et où le groupe phényle peut être substitué jusqu'à deux fois par des substituants Cl, F, CN, CF₃, alkyle en C₁-C₃, OH, O-alkyle en C₁-C₆ ;
R3 représente un atome de H, des groupes alkyle en C₁-C₆, F, (CH₂)ₙ-phényle, n peut être n = 0-5 et où le cycle phényle peut être substitué jusqu'à deux fois par des substituants Cl, F, CN, CF₃, alkyle en C₁-C₃, OH, O-alkyle en C₁-C₆, COO-alkyle en C₁-C₆, C(O)OH, C(O)NH₂ ;
R4 représente un groupe (CH₂)ₙ-R₅, n peut être n = 0-6 ;
R5 représente des groupes phényle, 1- ou 2-naphtyle
et R5 est substitué par des substituants NH-SO₂-alkyle en C₁-C₆, NH-SO₂-phényle, et où le groupe phényle peut être substitué jusqu'à deux fois par des substituants F, Cl, CN, OH, alkyle en C₁-C₆, O-alkyle en C₁-C₆, CF₃, COOH, COO-alkyle en C₁-C₆, CONH₂ ;
(CH₂)ₙ-SO₂-alkyle en C₁-C₆, n peut être égal à 1-6,
(CH₂)ₘ-SO₂-NH₂, (CH₂)ₘ-SO₂-NH-alkyle en C₁-C₆, (CH₂)ₘ-SO₂-N(alkyle en C₁-C₆)₂ ou (CH₂)ₘ-SO₂-N(=CH-N(CH₃)₂), m peut être m = 0-6 ;
et R₅ peut éventuellement être substitué en plus par des substituants F, Cl, Br, OH, CF₃, CN, OCF₃, O-alkyle en C₁-C₆, alkyle en C₁-C₆, COOH, COO-alkyle en C₁-C₆, CONH₂, NH₂, NH-CO-alkyle en C₁-C₆, NH-CO-phényle, (CH₂)ₙ-phényle, O-(CH₂)ₙ-phényle, SO₂-(CH₂)ₙ-phényle, n peut être n = 0-3 ;
ainsi que leurs sels physiologiquement tolérés.

4. Médicament contenant un ou plusieurs des composés selon une ou plusieurs des revendications 1 à 3.

5. Médicament contenant un ou plusieurs composés selon une ou plusieurs des revendications 1 à 3 et un ou plusieurs principes actifs anorectiques.

6. Composés selon une ou plusieurs des revendications 1 à 3 pour l'application comme médicament pour la prévention ou le traitement de l'obésité.

7. Composés selon une ou plusieurs des revendications 1 à 3 pour l'application comme médicament pour la prévention ou le traitement du diabète type II.

8. Composés selon une ou plusieurs des revendications 1 à 3 en combinaison avec au moins un autre principe actif anorectique pour l'application comme médicament pour la prévention ou le traitement de l'obésité.

9. Composés selon une ou plusieurs des revendications 1 à 3 en combinaison avec au moins un autre principe actif anorectique pour l'application comme médicament pour la prévention ou le traitement du diabète type II.

10. Procédé pour la préparation d'un médicament contenant un ou plusieurs des composés selon une ou plusieurs des revendications 1 à 3, **caractérisé en ce qu'**on mélange le principe actif avec un véhicule pharmaceutique approprié et on met ce mélange en forme appropriée pour l'administration.

11. Utilisation des composés selon une ou plusieurs des revendications 1 à 3 pour la préparation d'un médicament pour la prévention ou le traitement de l'obésité.

12. Utilisation des composés selon une ou plusieurs des revendications 1 à 3 pour la préparation d'un médicament pour la prévention ou le traitement du diabète de type II.

13. Procédé pour la préparation des composés selon une ou plusieurs des revendications 1 à 3, **caractérisé par** le schéma décrit par les formules suivantes : 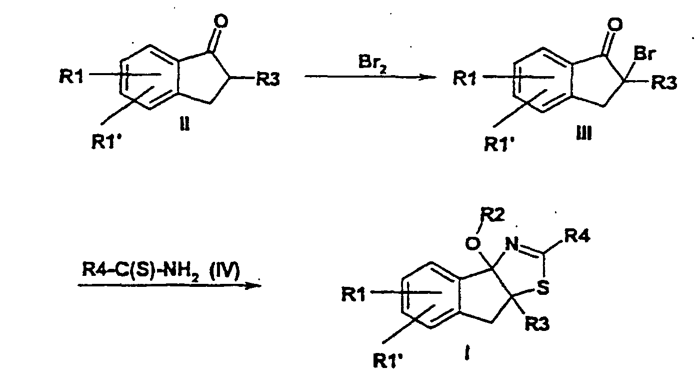 en faisant réagir un composé de formule II, où R1 et R1' et R3 possèdent les significations données à la formule I, sur du brome pour obtenir un composé III dans lequel R1, R1' et R3 possèdent les significations données à la formule I, et en faisant réagir ensuite le composé de formule III sur des thioamides de formule IV, où R4 possède la signification donnée à la formule I, pour obtenir un composé de formule I.
